Europäisches Patentamt

European Patent Office

Office européen des brevets

(19)

(11) Veröffentlichungsnummer: **0 041 610**
**B1**

(12) **EUROPÄISCHE PATENTSCHRIFT**

(45) Veröffentlichungstag der Patentschrift:
10.10.84

(21) Anmeldenummer: 81103251.5

(22) Anmeldetag: 30.04.81

(51) Int. Cl.³: **C 12 N 11/18, C 12 N 11/16,**
**C 12 P 1/00 // A21D8/00,**
**C12C11/00, C12H1/00**

(54) Coimmobilisate aus gärfähigen Hefen mit angekoppelten Enzymen sowie ihre Herstellung und Verwendung.

(30) Priorität: 09.06.80 DE 3021629

(43) Veröffentlichungstag der Anmeldung:
16.12.81 Patentblatt 81/50

(45) Bekanntmachung des Hinweises auf die Patenterteilung:
10.10.84 Patentblatt 84/41

(84) Benannte Vertragsstaaten:
AT BE CH DE FR IT LI LU NL SE

(56) Entgegenhaltungen:
DE - A - 2 410 693
FR - A - 2 276 318

NATURE, Band 235, 18. Februar 1972, Seite 389, London,
G.B. J.S. HOUGH et al.: "Couplings of enzymes onto microorganisms
CHEMICAL ABSTRACTS, Band 94, Nr. 15, 13. April 1981,
Seite 530, Nr. 119457k, Columbus, Ohio, USA B.
HAEGERDAL et al.: "The production of ethanol from cellobiose using baker's yeast co-immobilized with beta-glucosidase"

(73) Patentinhaber: C.H. BOEHRINGER SOHN, Postfach 200,
D-6507 Ingelheim am Rhein (DE)

(72) Erfinder: Hartmeier, Winfried, Leipzigerstrasse 3,
D-6507 Ingelheim/Rhein (DE)

## Beschreibung

Die Erfindung betrifft Coimmobilisate aus lebenden gärfähigen Hefezellen, insbesondere solche der Gattung Saccharomyces, an die aktive Enzyme angekoppelt sind, die von Natur aus nicht oder in für den avisierten Einsatzzweck nicht ausreichendem Maße in den betreffenden Hefezellen selbst vorhanden sind. Weiterhin betrifft die Erfindung die Herstellung dieser Coimmobilisate, die aus lebenden gärfähigen Hefezellen und gebundenen aktiven Enzymen bestehen. Schließlich betrifft die Erfindung die Verwendung der Enzym-Hefe-Coimmobilisate zur Durchführung biotechnischer Reaktionen, insbesondere zur alkoholischen Gärung.

Gärfähige Hefen werden seit Jahrtausenden von der Menschheit genutzt, um alkoholische Getränke zu bereiten. Dabei dient die Enzymausstattung der betreffenden Hefen dem Abbau einzelner oder mehrerer Komponenten des Substrates (z. B. vergärbarer Zucker in Bierwürze, Traubenmost, Brennereimaische) und dessen zumindest teilweiser Überführung in Äthylalkohol, der neben seiner klassischen Bedeutung als Genußmittel auch zunehmende Wichtigkeit als chemischer Grundstoff sowie als Treibstoff für Verbrennungsmotoren gewinnt.

Leider ist die Enzymausstattung der verwendeten Hefen nicht immer ausreichend, um alle erwünschten Reaktionen ablaufen zu lassen. Beispielsweise haben Weinhefen nur ungenügende proteinspaltende Aktivität, so daß Proteine im Most nur im geringen Umfang abgebaut werden. Dies kann zum Überschäumen der Gäransätze sowie zu erheblichen Klär- und Stabilitätsschwierigkeiten für die Weine führen, die nur durch zusätzliche Anwendung z. B. von Bentonit und anderen Schönungsmitteln (wie z. B. Gelatine und Kieselsol) behoben werden können.

Bisher ist auch nicht möglich, mit den üblichen Brennerei- und obergärigen Brauereihefen vom Typ Saccharomyces cerevisiae, das Trisaccharid Raffinose, das in vielen Rohstoffen der Brennerei vorkommt, zu vergären. Da diese Hefen $\alpha$-Galactosidase ( = Melibiase) fehlt, vergären sie Raffinose nur zu einem Drittel, die übrigen aus Melibiose bestehenden zwei Drittel der z. B. in Bierwürze oder Melasse vorkommenden Raffinose bleiben ungenutzt, so daß die Alkoholausbeute unter ihrem theoretisch möglichen Wert bleibt.

Für die Brauereipraxis mit untergärigen und auch obergärigen Bierhefen (Saccharomyces uvarum bzw. carlsbergensis und Saccharomyces cerevisiae) ist es nachteilig, daß diese Hefearten von Natur aus praktisch keine $\beta$-Glucanase enthalten, so daß $\beta$-Glucane der Bierwürze kaum gespalten werden und beim späteren Filtrieren des Bieres Schwierigkeiten bereiten. Analog verhält es sich mit dem Mangel der Weinhefen (Saccharomyces cerevisiae, Saccharomyces bayanus) an Pectinasen.

Es ist bereits Stand der Technik, oder zumindest durch den Stand der Technik nahegelegt, all jene Enzyme einem Gäransatz zuzusetzen, die die betreffende im Gäransatz befindliche Hefe nicht enthält. So verwendet man z. B. lösliche $\beta$-Glucanasen um einen hinreichenden Glucanabbau in Bier zu erreichen. Bei der Weinbearbeitung verwendet man bereits lösliche Pectinasen. Diese Arbeitsweisen haben jedoch den Nachteil, daß auch das fertige Getränk (z. B. Bier, Wein) noch das Enzymprotein enthält. Weitere Nachteile der Verwendung löslicher Enzyme sind z. B. im Falle löslichen Pepsins bei der Mostvergärung eine nur ungenügende Wirksamkeit hinsichtlich Schaumverhinderung.

Beim Einsatz der inzwischen zum allgemeinen Stand der Technik gehörenden immobilisierten Enzyme kann zwar das Endprodukt (z. B. Bier oder Wein) frei von Enzymprotein gehalten werden, diese Präparate führen aber — dem Gäransatz neben der Hefe zugesetzt — keineswegs zum gewünschten Erfolg eines schnellen Abbaus der von der gärenden Hefe nicht abbaubaren Substanz, es sei denn der Gäransatz wird mittels Rührer intensiv gerührt. Der erfolgreiche Einsatz der bekannten immobilisierten Präparate erfordert also einen zusätzlichen technischen Aufwand, der mit erheblichen Schwierigkeiten verbunden sein kann und in vielen Betrieben (Brauereien, Brennereien, Weinbaubetrieben) nicht durchführbar ist und sich oft auch wegen schädlicher Nebenwirkungen z. B. durch unerwünschten Sauerstoffeintrag und zu starken Hefezuwachs verbietet.

Es ist auch bereits durch Arbeiten von Y. Takasaki bekannt, daß man Enzyme an Mikroorganismenzellen binden kann. US-Patent Nr. 3 950 220 sowie Agr. Biol. Chem. 38, 1081—1082 (1974) geben Einzelheiten hierzu. Das Verfahren von Takasaki eignet sich für Schimmelpilze. Für gärende Hefezellen ist es nicht geeignet, da es zur völligen Inaktivierung des Gärenzymsystems der Zellen führt. Weiterhin hat es den Nachteil, daß nur relativ wenig Enzymprotein auf eine Zelle gebunden werden kann, die spezifische Enzymaktivität dieser Präparate also klein ist.

Der letztgenannte Nachteil geringer Enzym-Belegungsdichte auf den Enzym-Zell-Coimmobilisaten wird durch eine neue Methode von B. Hägerdal & K. Mosbach (Abstracts zum Food Process Engineering Congress, Helsinki, 1979) behoben, bei der $\beta$-Glucosidase in Alginatgel um die Zellen gehüllt wird. Diese Methode funktioniert mit lebenden gärfähigen Hefezellen, sie hat jedoch den Nachteil, daß sehr große Enzym-Hefezellpartikel entstehen, so daß die übliche Schwebefähigkeit der Hefezellen in zu vergärenden Medien (Maische, Würze, Most u. ä.) nicht mehr gegeben ist und erhebliche Minderungen der Gärleistung eintreten. Ein weiterer Nachteil ist der erhöhte Diffusionswiderstand, den die relativ dicke Enzym-Alginatschicht dem Durchtritt z. B. von vergärbaren Substanzen zur Hefezelle hin entgegengesetzt.

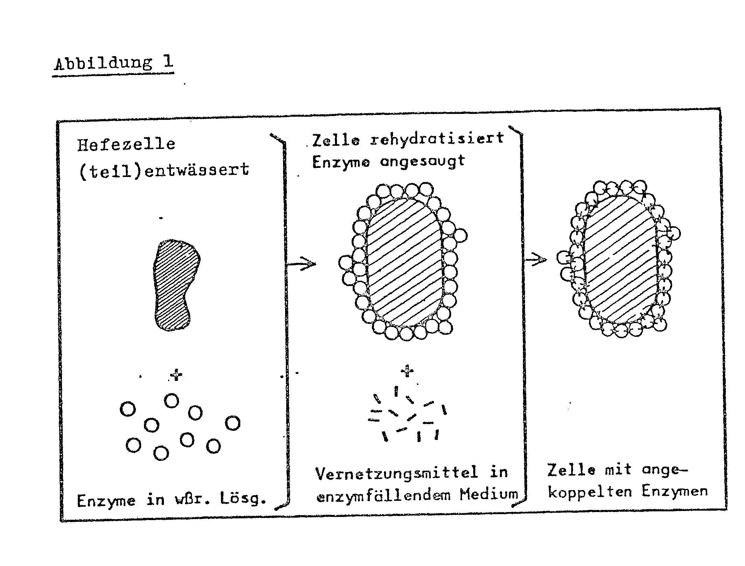

Es wurde nun gefunden, daß Präparate mit technischen und wirtschaftlichen Vorteilen entstehen, wenn man an gärfähige Hefezellen in der im Nachfolgenden näher erläuterten Weise unter Erhaltung der Gärfähigkeit Enzyme ankoppelt, die die betreffenden Hefen von Natur aus nicht oder in — für den avisierten Anwendungszweck — nicht ausreichendem Maße enthält. Präparate gemäß der Erfindung sind also Coimmobilisate aus im wesentlichen einzeln liegenden — also nicht agglomerierten — gärfähigen Hefezellen und einem oder mehreren Enzymen. Abbildung 1 erläutert den Grundaufbau eines derartigen Coimmobilisates sowie das Verfahren zur Herstellung desselben.

Gemäß der Erfindung werden die gärfähigen Hefezellen, die zumindest teilweise entwässert sein müssen, in eine wäßrige Lösung mit einem oder mehreren zur Ankopplung vorgesehenen Enzym eingebracht. Unter Wasseraufnahme schwellen die Hefezellen an und die Enzymmoleküle kommen bevorzugt auf die Hefezellen zu liegen. Der anschließende Zusatz einer enzymfällenden, aber nicht die Gärfähigkeit der Hefe inaktivierenden Lösung bewirkt, daß die Enzyme auf den Hefezellen haften bleiben bis durch Zugabe von enzymvernetzendem Agens die Enzymmoleküle untereinander fest verkettet sind und mit der gärfähigen Hefe das erfindungsgemäße Coimmobilisat bilden. Weiterhin hat der Zusatz der enzymfällenden Substanz den Effekt, daß die Hefezellen mit der sie umgebenden Enzymschicht vorwiegend einzeln zu liegen kommen und nicht untereinander zu größeren Aggregaten verbacken.

Die zumindest teilweise Entwässerung der eingesetzten Hefezellen kann — sofern sie nicht bereits vorgegeben ist — nach einem an sich bekannten Verfahren zur Herstellung aktiver Trockenhefe (z. B. nach dem in der DE-PS 2 515 029 angegebenen Vakuumtrocknungsverfahren) erfolgen. Eine weitere bekannte und gut geeignete Methode zum zumindest teilweisen Wasserentzug aus dem Zellinnern ist die Anwendung von Substanzlösungen (z. B. Salzen, Zucker, Glycerin) mit osmotisch hohem Druck. Die Zellen geben bei Einbringung in derartige Lösungen mit hohem osmotischem Wert einen Teil ihres Wassers aus dem Zellinnern in die umgebende Lösung ab und sind nach Abtrennung der umgebenden Lösung als nunmehr teilentwässertes Ausgangs-Zellmaterial zur Herstellung des erfindungsgemäßen Hefe-Enzym-Coimmobilisates geeignet.

Die mehr oder weniger entwässerten Hefezellen werden gemäß der Erfindung in wäßrige Enzymlösung eingebracht. Die Wassermenge der Enzymlösung ist dabei so zu bemessen, daß die Hefezellen nach ihrer Rehydratisierung das Wasser zum größten Teil aufnehmen und möglichst wenig oder kein Wasser zwischen den Zellen verbleibt. Die erforderliche Wassermenge kann in Vorversuchen ermittelt werden. Überschlagsmäßig sollte sie so groß sein, daß ihr völliges Aufsaugen den Wassergehalt der Hefezellen in die Größenordnung von 70 bis 75% bringt. Während der Rehydratisierung der Hefezellen in der wäßrigen Enzymlösung werden die Enzyme an die Hefezellen herangezogen und kommen auf deren Zelloberfläche zu liegen.

Bei der Rehydratisierung (= Wiederbefeuchtung) muß bekanntermaßen vorsichtig vorgegangen werden, da Hefezellen allgemein um so empfindlicher z. B. gegen Kälteschock bei der Rehydratisierung sind, je stärker ihr Trocknungsgrad ist. Es ist deshalb zweckmäßig, die Wiederbefeuchtung mit auf ca. 30 bis 40°C angewärmter wäßriger Enzymlösung vorzunehmen. Gewünschtenfalls können den Hefezellen auch entsprechende Schutzstoffe, wie es z. B. die DE-OS 2 531 800 und DE-OS 2 435 226 vorzusehen, zugemischt sein, die die Rehydratisierung erleichtern bzw. die Zellen vor Schädigung ihrer Gärfähigkeit schützen.

Als Vernetzungsmittel können allgemein bekannte bi- oder polyfunktionelle Verbindungen — wie z. B. Diisocyanate, Glutardialdehyd, Hexamethylendiamin oder Hexamethylentetramin — eingesetzt werden. Bevorzugt wird Glutardialdehyd in wäßriger Lösung einer Konzentration von 1—10% in der Reaktionsmischung verwendet. Die Vernetzung wird wenige Minuten bis zu mehrere Tage lang, bevorzugt 1—5 Stunden lang bei 20 bis 30°C durchgeführt. Dabei ist es günstig, den Reaktionsansatz z. B. durch Rühren oder Schütteln etwas zu bewegen. Nach Beendigung wird das entsprechende Präparat in der Regel gewaschen und im feuchten oder gewünschtenfalls getrockneten Zustand seiner weiteren Verwendung zugeführt.

Tabelle 1 gibt einige Beispiele für erfindungsgemäße Kombinationen von Hefezellen und Enzymen und deren wirtschaftlich-technische Verwendungsmöglichkeiten.

Als besonders vorteilhaft hat sich die Kopplung von Weinhefe der Typen Saccharomyces cerevisiae und Saccharomyces bayanus mit Pepsin erwiesen. Überraschenderweise ermöglichen die erfindungsgemäßen Coimmobilisate bei Einsatz in eiweißreichen Mosten eine praktisch schaumfreie Vergärung, die bei Einsatz von löslichem Pepsin in gleicher Menge neben normaler Hefe gleicher Menge nicht erfolgt. Weiterhin bringt der Einsatz des erfindungsgemäßen Präparates eine beschleunigte Gärung, eine schnellere Selbstklärung der Weine sowie eine Ersparnis an Mitteln zur Schönung der Weine.

Tabelle 1

| Hefeart | angekoppeltes Enzym | Verwendungsmöglichkeit |
|---|---|---|
| Saccharomyces cerevisiae oder bayanus | Pepsin | Weinherstellung aus eiweißreichen Mosten |
| Saccharomyces cerevisiae oder bayanus | Pectinase | Frucht- und Traubenmostvergärung |
| Saccharomyces cerevisia | $\beta$-Galactosidase (= Lactase) | Vergärung von Molke u. a. lactosehaltigen Medien |
| Saccharomyces cerevisiae | $\alpha$-Amylase/Amyloglucosidase | Vergärung stärke- oder dextrinhaltiger Medien |
| Saccharomyces cerevisiae | $\alpha$-Galactosidase | Vergärung raffinosehaltiger Maischen |
| Saccharomyces cerevisiae | Cellulose/Cellobiase | Vergärung cellulosehaltiger Maischen |
| Saccharomyces uvarum | Papain | Herstellung eiweißstabiler Biere |
| Saccharomyces uvarum | $\beta$-Glucanase | Bierbereitung aus glucanreichen Würzen |
| Saccharomyces uvarum | $\alpha$-Amylase/Amylo-glocusidase | Diätbierherstellung |
| Kloeckera appiculata | Pepsin | Angärung von Weinmost mit hohem Sulfitgehalt |

Die nachfolgenden Beispiele sollen die Erfindung erläutern ohne sie einzuschränken.

Beispiel 1

14 g frische Bäckerei-Preßhefe von der Hefefabrik Pleser, Darmstadt wurden mit 1,4 g pulvrigem Sorbit ca. 15 Minuten lang verrührt. Dabei wurde die Hefe durch Abgabe von intrazellulärem Wasser (aus dem Zellinnern) in den interzellulären Raum (Raum zwischen den Zellen) teilentwässert. Die Hefe wurde dann auf einer Filternutsche abgesaugt, so daß die von den Zellen abgegebene Flüssigkeit mit dem zugesetzten Sorbit weitgehend entfernt wurde. Die Hefe hatte nach dieser Entwässerungsprozedur nur noch 47% Wassergehalt bzw. 53% Trockensubstanzgehalt.

Getrennt zum o. a. Entwässerungsansatz wurden 0,5 g des handelsüblichen cellulolytischen Enzympräparates »Cellulase AP3« von der Firma Amano Pharmaceutical Co. Ltd., Nagoya (Japan) bei Raumtemperatur mit 5 ml entionisiertem Wasser gelöst. In diese Enzymlösung wurde die gesamte — gemäß obiger Beschreibung — teilentwässerte Preßhefe eingerührt. Es wurde 15 Minuten lang weitergerührt bis die Hefe gut rehydratisiert und in Suspension gegangen war. Der Ansatz wurde dann auf 25°C temperiert, mit 20 ml 2%iger Tanninlösung und 0,1 ml 25%iger Glutardialdehydlösung versetzt. Nach der Aldehydzugabe wurde der Ansatz in einem Erlenmeyerkolben 4 Stunden lang bei 25°C geschüttelt. Danach wurde das entstandene Enzym-Hefe-Coimmobilisat gründlich mit Wasser gewaschen.

Zur Prüfung der Gärfähigkeit des Coimmobilisates wurde dieses in einem 50-ml-Gäransatz mit 1 g Cellobiose und 0,1 g $KH_2PO_4$ überführt, dessen pH-Wert auf 5,0 eingestellt war. Der Gäransatz wurde bei 30°C temperiert und die $CO_2$-Entwicklung gemessen. Sie betrug 30 ml nach 1 Stunde, 80 ml nach 2 Stunden und 140 ml nach 3 Stunden.

Die starke $CO_2$-Entwicklung zeigt die gute Vergärungsleistung des erfindungsgemäßen Coimmobilisates gegenüber Cellobiose, die sonst von Backhefen überhaupt nicht vergoren wird.

## Beispiel 2

0,5 Liter dickbreiige untergärige Bierhefe (Saccharomyces uvarum) wurden mit 100 ml Glycerin 30 Minuten lang gerührt. Danach wurde die überstehende Flüssigkeit über eine Filternutsche abgesaugt. Der entstandene Heferückstand hatte einen Wassergehalt von 49% bzw. einen Trockensubstanzgehalt von 51%. Diese gepreßte, teilentwässerte Feuchthefe diente als Ausgangshefe für die u. a. Versuche zur Coimmobilisierung mit Amyloglucosidase und die Vergärung von Bierwürze.

0,5 g der handelsüblichen Amyloglucosidase (= Glucoamylase) »Glucozyme 8000« von der Firma Amano Pharmaceutical Co. Ltd., Nagoya (Japan) wurden bei Raumtemperatur mit 5 ml entionisiertem Wasser gelöst. In diese Enzymlösung wurden 10 g der oben beschriebenen angepreßten, teilentwässerten Bierhefe eingerührt. Nach 15minütigem Weiterrühren wurden 20 ml 1%ige Tanninlösung und 0,15 ml 25%ige Glutardialdehydlösung hinzugemischt. Dieser Ansatz wurde 2 h im Erlenmeyerkolben bei 25°C geschüttelt. Danach wurde das entstandene Enzym-Hefe-Coimmobilisat gründlich mit Wasser gewaschen. Das überschüssige Wasser wurde schließlich über eine Nutsche abgesaugt.

Unter Verwendung frischer heller Ausschlagwürze aus einer Brauerei wurde in üblicher Weise nach der sog. »Normalen Methode« (Pawlowski-Schild: Die brautechnischen Untersuchungsmethoden S. 165—167, 8. Aufl., Verlag Carl, Nürnberg) der scheinbare Endvergärungsgrad bestimmt. Es ergab sich bei Einsatz der teilentwässerten Bierhefe ein scheinbarer Endvergärungsgrad von 80%, während bei Einsatz der mit Amyloglucosidase coimmobilisierten Hefe 102% erreicht wurden. Die coimmobilisierte Hefe führt also zu einem Wert, wie er für Diätbiere typisch ist.

## Beispiel 3

0,5 g der handelsüblichen Pilzlactase »Galantase« aus Aspergillus niger von der Firma Tokyo Tanabe Co. Ltd., Tokyo (Japan) wurden mit 5 ml entionisiertem Wasser gelöst. Die Lösung wurde dann auf 35°C erwärmt und es wurden 2 g einer handelsüblichen Trockenbackhefe von der Firma Dr. Oetker, Bielefeld hinzugerührt. Nach 15minütigem Weiterrühren war die Trockenhefe gut rehydratisiert und suspendiert. Der entstandene teigige Hefebrei wurde auf 25°C abgekühlt, mit 20 ml 2%iger Tanninlösung und 0,05 ml 25%iger Glutardialdehydlösung und 25 mg Hexamethylentetramin versetzt. Der gesamte Ansatz wurde dann im Erlenmeyerkolben 2 Stunden lang bei 25°C im Schüttelwasserbad geschüttelt. Danach wurde das entstandene Lactase-Hefe-Coimmobilisat gründlich mit entionisiertem Wasser gewaschen. In 100 ml einer 5%igen Lactoselösung, die mit 0,1% Na-Citrat versetzt und auf pH 4,5 eingestellt war, entwickelte das Coimmobilisat bei 30°C nach 30 Minuten 130 ml $CO_2$ nach 60 Minuten 210 ml $CO_2$. Das Coimmobilisat hatte damit beträchtliche Gärfähigkeit gegenüber Lactose, die für normale Backhefe nicht vergärbar ist.

## Beispiel 4

0,5 g eines handelsüblichen Schweinepepsins mit 2000 FIP-units/g (von der Firma Merck, Darmstadt, Art. Nr. 7190) wurden mit 6 ml entionisiertem Wasser gelöst. Nach der Auflösung des Pepsins wurde die Lösung auf 38°C erwärmt und es wurden 2 g der handelsüblichen Trockenweinhefe »Irgaferm CM« (= Handelsprodukt der Anmelderin) hinzugerührt. Nach 15minütigem Rühren war die Trockenhefe klumpenfrei rehydratisiert. Der teigige Hefebrei wurde auf 25°C heruntergekühlt und mit 20 ml 1%iger Tanninlösung und 0,1 ml 25%iger Glutardialdehydlösung versetzt. Der gesamte Ansatz wurde dann im Erlenmeyerkolben 2 Stunden lang bei 25°C geschüttelt. Danach wurde das entstandene Pepsin-Weinhefe-Coimmobilisat gründlich mit Leitungswasser gewaschen.

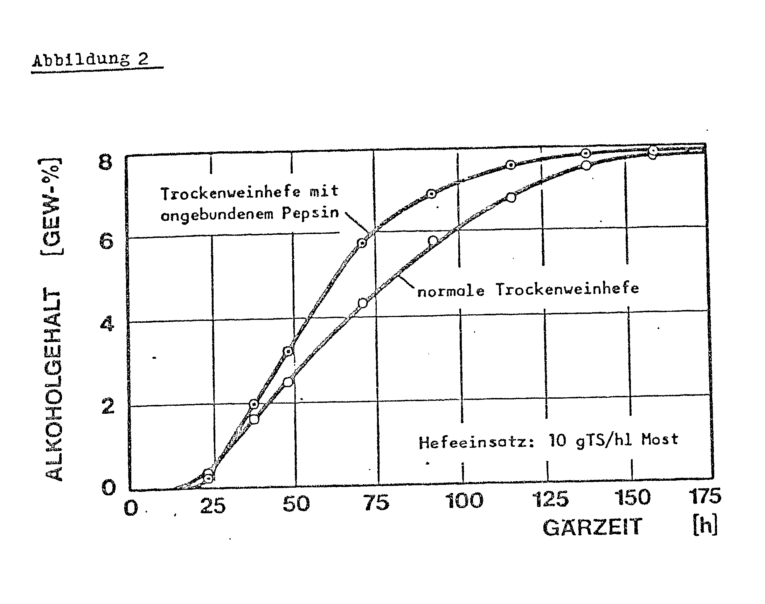

Die Gesamtmenge des gerade beschriebenen Pepsin-Weinhefe-Coimmobilisates wurde zu 10 Liter frischem Silvaner-Traubenmost zur Einleitung der Gärung bei 20°C zugesetzt. Parallel dazu wurden 2 g nicht mit Pepsin coimmobilisierte Trockenweinhefe »Irgaferm« nach Rehydratisierung bei 38°C zu 10 Liter desselben Silvaner-Traubenmostes zugesetzt. Es zeigt sich, daß die Gärung mit dem erfindungsgemäßen Coimmobilisat selbst in der intensivsten Phase nie über 1 cm Schaumdecke zeigte, während die Schaumdecke der Kontrolle nahezu das Volumen der Gärflüssigkeit erreichte. Als weiterer Vorteil des erfindungsgemäßen Coimmobilisates ergab sich eine schnellere Vergärung des Mostes. Abbildung 2 verdeutlicht dies. Weiterhin wurde bei Verwendung des erfindungsgemäßen Produktes eine bessere Selbstklärung des Weines beobachtet und es wurde ein eiweißstabiler Wein ohne weitere Schönung (etwa durch Betonit, Gelatine, Kieselsol) erzielt. Letzteres zeigte sich deutlich, wenn man die beiden filtrierten Weinproben 24 h auf 60°C erwärmte und wieder auf 20°C abkühlte. Der mit dem erfindungsgemäßen Präparat hergestellte Wein blieb bei dieser Behandlung glanzklar, während der Kontrollwein starke Trübung zeigte.

**0 041 610**

## Patentansprüche

1. Coimmobilisate aus Hefe mit angekoppelten Enzymen, dadurch gekennzeichnet, daß das Enzym oder ein Enzymgemisch unmittelbar umhüllend an lebende gärfähige Hefezellen angekoppelt ist.

2. Coimmobilisate nach Anspruch 1, dadurch gekennzeichnet, daß die Kopplung von Enzym und Hefe mittels an sich bekannter Vernetzungsmittel bewirkt ist.

3. Coimmobilisate nach einem der Ansprüche 1 oder 2, dadurch gekennzeichnet, daß die Kopplung mittels Glutardialdehyd bewirkt ist.

4. Coimmobilisate nach einem der vorhergehenden Ansprüche, dadurch gekennzeichnet, daß als Hefe Weinhefe vom Typ Saccharomyces cerevisiae oder Saccharomyces bayanus und als Enzym Pepsin eingesetzt worden sind.

5. Coimmobilisate nach Anspruch 1, dadurch gekennzeichnet, daß im wesentlichen einzelne Hefezellen von einem Enzym oder Enzymgemisch unmittelbar umhüllend umgeben sind.

6. Verfahren zur Herstellung von Coimmobilisaten nach Anspruch 1, dadurch gekennzeichnet, daß Hefezellen ganz oder teilweise entwässert, anschließend mittels einer wäßrigen Enzymlösung rehydratisiert, ein die Gärfähigkeit der Hefezellen nicht beeinträchtigendes Enzymfällungsmittel und schließlich ein an sich bekanntes Vernetzungsmittel zugesetzt wird.

7. Verfahren nach Anspruch 6, dadurch gekennzeichnet, daß als Hefe Saccharomyces cerevisiae oder Saccharomyces bayanus und als Enzym Pepsin eingesetzt wird.

8. Verfahren nach Anspruch 6 oder 7, dadurch gekennzeichnet, daß die Rehydratisierung mittels einer wäßrigen Enzymlösung erfolgt, deren Wassermenge so bemessen ist, daß Hefezellen das Wasser nahezu gänzlich aufnehmen können.

9. Verfahren nach einem der Ansprüche 6 bis 8, dadurch gekennzeichnet, daß die Rehydratisierung mittels auf ca. 30—40° C angewärmter Enzymlösung erfolgt.

10. Verfahren nach Anspruch 6, dadurch gekennzeichnet, daß als Enzymfällungsmittel Tannin eingesetzt wird.

11. Verfahren nach Anspruch 6, dadurch gekennzeichnet, daß als Vernetzungsmittel Glutardialdehyd eingesetzt wird.

12. Verwendung von Enzympräparaten nach einem der Ansprüche 1 bis 5 zur Durchführung biotechnischer Verfahren.

## Claims

1. Co-immobilisates of yeast with enzymes coupled to them, characterised in that the enzyme or an enzyme mixture is coupled to living yeast cells capable of fermentation so as to surround them directly.

2. Co-immobilisates as claimed in claim 1, characterised in that the coupling of the enzymes and yeast is effected by means of cross-linking agents known per se.

3. Co-immobilisates as claimed in one of claims 1 and 2, characterised in that the coupling is effected by means of glutardialdehyde.

4. Co-immobilisates as claimed in one of the preceding claims, characterised in that the yeast used is wine yeast of the Saccharomyces cerevisiae or Saccharomyces bayanus type and the enzyme used is pectin.

5. Co-immobilisates as claimed in claim 1, characterised in that substantially individual yeast cells are surrounded by an enzyme or mixture of enzymes so as to enclose them directly.

6. Process for producing co-immobilisates as claimed in claim 1, characterised in that yeast cells are wholly or partially dewatered, then rehydrated by means of an aqueous enzyme solution, an enzyme precipitating agent which does not affect the fermentation of the yeast cells is added and finally a cross-linking agent known per se is added.

7. Process as claimed in claim 6, characterised in that Saccharomyces cerevisiae or Saccharomyces bayanus is used as the yeast and pectin is used as the enzyme.

8. Process as claimed in claim 6 or 7, characterised in that the rehydration is effected by means of an aqueous enzyme solution the water content of which is such that cells can take up almost all the water.

9. Process as claimed in one of claims 6 to 8, characterised in that the rehydration is effected by means of an enzyme solution heated to about 30 to 40° C.

10. Process as claimed in claim 6, characterised in that tannin is used as the enzyme precipitating agent.

11. Process as claimed in claim 6, characterised in that glutardialdehyde is used as the crosslinking agent.

12. Use of enzyme preparations as claimed in one of claims 1 to 5 for carrying out biotechnical processes.

6

0 041 610

## Revendications

1. Coimmobilisats de levure avec des enzymes couplées, caractérisés en ce que l'enzyme ou un mélange d'enzymes est couplé en les enveloppant directement sur des cellules de levure vivantes capables de réaliser des fermentations.

2. Coimmobilisats suivants la revendication 1, caractérises en ce que le couplage de l'enzyme et de la levure est réalisé au moyen d'agents réticulants connus en soi.

3. Coimmobilisats suivant l'une des revendications 1 ou 2, caractérisés en ce que le couplage est réalisé au moyen de glutardialdéhyde.

4. Coimmobilisats suivant l'une des revendications précédentes, caractérisés en ce qu'on a utilisé comme levure de la levure de vin du type Saccharomyces cerevisiae ou Saccharomyces bayanus et comme enzyme de la pepsine.

5. Coimmobilisats suivant la revendication 1, caractérisés en ce que des cellules de levure essentiellement isolées sont entourées d'une enzyme ou d'un mélange d'enzymes les enveloppant directement.

6. Procédé de préparation de coimmobilisats suivant la revendication 1, caractérisé en ce qu'aux cellules de levure totalement ou partiellement déshydratées, puis réhydratées au moyen d'une solution aqueuse d'enzyme, on ajoute un agent de précipitation de l'enzyme n'affectant pas la capacité de réaliser des fermentations des cellules de levure, et enfin un agent de réticulation connu en soi.

7. Procédé suivant la revendication 6, caractérisé en ce qu'on utilise comme levure Saccharomyces cerevisiae ou Saccharomyces bayanus et comme enzyme de la pepsine.

8. Procédé suivant l'une des revendications 6 ou 7, caractérisé en ce que la réhydratation est effectuée au moyen d'une solution aqueuse d'enzyme dont la quantité d'eau est mesurée de telle façon que les cellules de levure peuvent fixer l'eau presque complètement.

9. Procédé suivant l'une des revendications 6 à 8, caractérisé en ce que la réhydration s'effectue au moyen d'une solution d'enzyme chauffée à environ 30—40° C.

10. Procédé suivant la revendication 6, caractérisé en ce qu'on utilise du tanin comme agent de précipitation de l'enzyme.

11. Procédé suivant la revendication 6, caractérisé en ce qu'on utilise comme agent de réticulation du glutardialdéhyde.

12. Utilisation de préparations d'enzymes suivant l'une des revendications 1 à 5 pour l'exécution de procédés biotechniques.

7

## Abbildung 1

**Hefezelle (teil)entwässert** — Enzyme in wßr. Lösg.

**Zelle rehydratisiert Enzyme angesaugt** — Vernetzungsmittel in enzymfällendem Medium

**Zelle mit angekoppelten Enzymen**

Abbildung 2